# EUROPEAN PATENT APPLICATION

(11) **EP 1 078 992 A2**
(43) Date of publication of application: **28.02.2001**
(21) Application number: 00307252.7
(22) Date of filing: 23.08.2000
(51) Int. Cl.: C12Q 1/68

(54) **Polyclonal and Monoclonal Oligobodies (Synthetic, Oligonucleotide-Based Antibody-Like Reagents), and a method of producing oligobodies**

(30) Priority: 26.08.1999 AR 9904272
(71) Applicant: Santa Coloma, Tomas Antonio, Buenos Aires (AR)
(72) Inventor: Santa Coloma, Tomas Antonio, Buenos Aires (AR)
(74) Representative: Daniels, Jeffrey Nicholas

(57) **Abstract**

Utilizing a library of oligonucleotides and synthetic peptides corresponding to fragments of different proteins, together with a strategy that we have called "target switching", we were successful in producing reagents equivalent to antibodies in their ability to recognize target proteins in both native and denatured form and useful in similar applications for which monoclonal or polyclonal antibodies are used. We have called "oligobodies" to these reagents.

SEQUENCE LISTING

<110> SANTA COLOMA, TOMAS A

<120> POLYCLONAL AND MONOCLONAL OLIGOBODIES (SYNTHETIC,
      OLIGONUCLEOTIDE-BASED ANTIBODY-LIKE REAGENTS), AND A
      METHOD OF PRODUCING OLIGOBODIES

<130> 102637

<140>
<141>

<150> P990104272
<151> 1999-08-26

<160> 8

<170> PatentIn Ver. 2.1

<210> 1
<211> 19
<212> DNA
<213> Artificial Sequence

<220>
<223> Description of Artificial Sequence:PRIMER

<400> 1
ctgcagccgc ggggatcct                                              19


<210> 2
<211> 25
<212> DNA
<213> Artificial Sequence

<220>
<223> Description of Artificial Sequence:PRIMER

<400> 2
gccactaagc ttgaattcga ctaga                                       25


<210> 3
<211> 45
<212> DNA
<213> Artificial Sequence

<220>
<223> Description of Artificial Sequence:PRIMER

<400> 3
ctgcagccgc ggggatccnt ctagtcgaat tacaagctta gtggc                45

<210> 4
<211> 12
<212> PRT
<213> Unknown Organism

<220>
<223> Description of Unknown Organism:RESIDUES 298-309
      OF THE CATALYTIC SUBUNIT OF PROTEIN PHOSPHATASE 2A

<400> 4
Pro His Val Thr Arg Arg Thr Pro Asp Tyr Phe Leu
  1               5                  10

<210> 5
<211> 13
<212> PRT
<213> Unknown Organism

<220>
<223> Description of Unknown Organism:RESIDUES 268-280
      OF THE CATALYTIC SUBUNIT OF PROTEIN PHOSPHATASE 2A

<400> 5
Arg Met Pro Pro Arg Arg Asp Ala Met Pro Ser Asp Ala
  1               5                  10

<210> 6
<211> 15
<212> PRT
<213> Artificial Sequence

<220>
<223> Description of Artificial Sequence:FRAGMENT OF
      PROTEIN CPD1 DEDUCED FROM CDNA SEQUENCE

<400> 6

Cys Pro Asn Leu Thr Tyr Leu Asp Leu Ser Gly Asn Lys Ile Lys
1               5                   10                  15

<210> 7
<211> 97
<212> DNA
<213> Artificial Sequence

<220>
<223> Description of Artificial Sequence:OLIGONUCLEOTIDE
      SEQUENCE SPECIFIC FOR PROTEIN CPD1

<400> 7
ctgcagccgc ggggatcctg gttagccccg aggactggtt tgcactgaca tgcgcgtgtg 60
gttggctact cgtctagtcg aattcaagct tagtggc                          97

<210> 8
<211> 19
<212> DNA
<213> Artificial Sequence

<220>
<223> Description of Artificial Sequence:PRIMER

<400> 8
ctgcagccgc ggggatcct                                              19

## Description

The present invention relates to synthetic antibody-like reagents, referred to hereinafter as "oligobodies" and to a method to prepare them efficiently. These reagents are constituted by double-strand or single-strand oligonucleotides selected from a combinatorial library of oligonucleotides, having very high specificity toward native or denatured proteins. These reagents have proved efficacy as a replacement for monoclonal or polyclonal antibodies in their different applications such as immunoblotting, immunohistochemistry and immunoprecipitations, characteristics not present in any synthetic reagent known in previous art.

In the detection, isolation and purification of proteins, the capacity of antibodies to recognize them either in native or denatured form is used. However, needed antibodies are not always readily available, especially monoclonal antibodies, since the procedures for obtaining and purifying these antibodies are generally time-consuming, and expensive. In addition, to develop antibodies, the molecule to be recognized by the immune system should be "antigenic"; in other words, it should be recognized by the immune system as a foreign protein, a situation not often occurring. On the other hand, antibodies are bulky, a property that not only affects their diffusion into tissues and their accessibility to targets such as tumors, but also might trigger immunogenic reactions. These factors, among others, affect and limit the therapeutic applications of antibodies.

It is of great interest, therefore, to be able to develop "synthetic antibodies", i.e., synthetic molecules obtained without a need for animals, mammalian cells, immune system, etc., which are capable of behaving like antibodies in assays such as Western blots, immunohistochemistry, immunoprecipitations, etc. In addition to be important tools for research and diagnosis, these molecules could also help to improve the efficiency of methods for immunotherapy in diseases such as cancer or in autoimmune processes.

Some recent methods have been conceived and applied to prepare reagents that recognize proteins with high affinity. These methods are based on the capacity of certain oligonucleotides (DNA or RNA) to bind to targets that are not oligonucleotides, such as proteins, small molecules, metabolites, etc. These reagents are constituted by populations of single-strand chains, having sequences derived from combinatorial synthesis that contain regions of random sequence, and obtained by adding in each step of the synthesis a mixture of the four bases A, C, G, and T. In this way, libraries or collections of single-strand oligonucleotides are obtained, having billons of different conformations (about 10¹⁴), some of them being able to bind to the target with high affinity. The initial work in this area was carried out by Tuerk and Gold (Tuerk and Gold (1990) "Systematic evolution of ligands by exponential enrichment: RNA ligands to bacteriophage T4 DNA polymerase" , *Science,* 249:505-10 ), Ellington and Szostak (Ellington and Szostak (1990) "In vitro selection of RNA molecules that bind specific ligands" , Nature, 346:818-22 ) and Blackwell and Weintraub (Blackwell and Weintraub (1990) "Differences and similarities in DNA-binding preferences of MyoD and E2A protein complexes revealed by binding site selection" , *Science,* 250:1104-10).

The procedure for selecting members of these libraries with affinity for certain targets was called SELEX ("systematic evolution of ligands by exponential enrichment") by Larry Gold and colleagues (Tuerk and Gold (1990) "Systematic evolution of ligands by exponential enrichment: RNA ligands to bacteriophage T4 DNA polymerase" , *Science,* 249:505-10 ); wile Ellington and colleagues called these reagents "aptamers" (Ellington and Szostak (1990) "In vitro selection of RNA molecules that bind specific ligands" , Nature, 346:818-22 ). Briefly, a mixture of oligonucleotides with a random core sequence is challenged with a target molecule generally bound to a solid support. After binding of those oligonucleotides with affinity for the target, it is possible to select and separate those having higher affinity by using washing steps, discarding oligonucleotides of low or null affinity. Then, the bound, high affinity oligonucleotides are separated from the target and PCR-amplified by using forward and reverse primers containing sequences complementary to the fixing sequences at both 5'and 3' ends of the library. This procedure results in multiple copies of double-stranded oligonucleotides. Then, these strands can be separated by using several different procedures and bound again to the target as single-stranded molecules of mdltiple conformations. By repeating this cycle several times, it is possible to increase the amount of aptamers in each step with high affinity for the target. Several variants of this general method are mentioned in the cited literature.

However, the results obtained by SELEX are not yet satisfactory. Even after 10 years of the initial investigations, only a limited amount of aptamers has been obtained towards approximately 150 different molecules. Examples include: thrombin (Bock et al. (1992) "Selection of single-stranded DNA molecules that bind and inhibit human thrombin" , *Nature,* 355:564-6 ), selectin P (Jenison et al. (1998) "Oligonucleotide inhibitors of P-selectin-dependent neutrophil-platelet adhesion" , *Antisense Nucleic Acid Drug Dev.,* 8:265-79 ), VEGF (Jellinek et al. (1994) "Inhibition of receptor binding by high-affinity RNA ligands to vascular endothelial growth factor" , *Biochemistry,* 33:10450-6 ; Ruckman et al. (1998) "2'-Fluoropyrimidine RNA-based aptamers to the 165-amino acid form of vascular endothelial growth factor (VEGF165). Inhibition of receptor binding and VEGF-induced vascular permeability through interactions requiring the exon 7-encoded domain", J. *Biol. Chem.,* 273:20556-67 ); Interferon γ (Balasubramanian et al. (1998) "Interferon-gamma-inhibitory oligodeoxynucleotides alter the conformation of interferon-gamma" , *Mol. Pharmacol.,* 53:926-32 ; Lee et al. (1996) "An oligonucleotide blocks interferon-gamma signal transduction" , *Transplantation,* 62:1297-301 ; Kubik et al. (1997) "Isolation and characterization of 2'-fluoro-, 2'-amino-, and 2'-fluoro- /amino-modified RNA ligands to human IFN-gamma that inhibit receptor binding" , *J. Immunol.,* 159:259-67 ); CD4 (Davis et al. (1998) "Staining of cell surface human CD4 with 2'-F-pyrimidine-containing RNA aptamers for flow cytometry" , *Nucleic Acids Res.,* 26:3915-24 ; Kraus et al. (1998) "Novel RNA ligands able to bind CD4 antigen and inhibit CD4+ T lymphocyte function" , *J. Immunol.,* 160:5209-12 ); subtilisin (Takeno et al. (1999) "Selection of an RNA molecule that specifically inhibits the protease activity of subtilisin" , *J. Biochem.,* 125:1115-9 ); protease NS3 from hepatitis (Kumar et al. (1997) "Isolation of RNA aptamers specific to the NS3 protein of hepatitis C virus from a pool of completely random RNA" , *Virology,* 237:270-82 ; Urvil et al. (1997) "Selection of RNA aptamers that bind specifically to the NS3 protease of hepatitis C virus" , *Eur.* J. *Biochem.,* 248:130-8 ); elastase (Davis et al. (1996) "Use of a high affinity DNA ligand in flow cytometry" , *Nucleic Acid Res.,* 24:702-6 ; Charlton et al. (1997) "In vivo imaging of inflammation using an aptamer inhibitor of human neutrophil elastase" , *Chem. Biol.,* 4:809-16 ; Bless et al. (1997) "Protective effects of an aptamer inhibitor of neutrophil elastase in lung inflammatory injury" , *Curr. Biol.,* 7:877-80 ); some proteins of the HIV-1 virus (Boiziau et al. (1997) "Identification of aptamers against the DNA template for in vitro transcription of the HIV-1 TAR element" , *Antisense Nucleic Acid Drug Dev.,* 7:369-80 ; Allen et al. (1996) "A specific RNA structural motif mediates high affinity binding by the HIV-1 nucleocapsid protein (NCp7)" , *Virology,* 225:306-15 ; Berglund et al. (1997) "A high affinity binding site for the HIV-1 nucleocapsid protein" , *Nucleic Acid Res.,* 25:1042-9 ; Boiziau et al. (1999) "DNA aptamers selected against the HIV-1 trans-activation-responsive RNA element form RNA-DNA kissing complexes" , *J. Biol. Chem.,* 274:12730-7 ; Giver et al. (1993) "Selection and design of high-affinity RNA ligands for HIV-1 Rev" , *Gene*, 137:19-24 ; Jensen et al. (1994) "Characterization of an in vitro-selected RNA ligand to the HIV-1 Rev protein" , *J. Mol. Biol.,* 235:237-47 ; Marozzi et al. (1998) "In vitro selection of HIV-1 TAR variants by the Tat protein" , *J. Biotechnol.,* 61:117-28 ; Schneider et al. (1995) "High-affinity ssDNA inhibitors of the reverse transcriptase of type 1 human immunodeficiency virus" , *Biochemistry,* 34:9599-610 ; Tuerk and MacDougal-Waugh (1993) "In vitro evolution of functional nucleic acids: high-affinity RNA ligands of HIV-1 proteins" , *Gene,* 137:33-9); small molecules such as adenosine (Burke and Gold (1997) "RNA aptamers to the adenosine moiety of S-adenosyl methionine: structural inferences from variations on a theme and the reproducibility of SELEX" , *Nucleic Acids Res.,* 25:2020-4 ; Huizenga and Szostak (1995) "A DNA aptamer that binds adenosine and ATP" , *Biochemistry,* 34:656-65 ), dopamine (Mannironi et al. (1997) "In vitro selection of dopamine RNA ligands" , *Biochemistry,* 36:9726-34 ) and others.

Utilizing a somewhat different strategy, Xu and Ellington applied SELEX to a synthetic peptide that corresponded to a fragment of the protein Rev (Xu and Ellington (1996) "Anti-peptide aptamers recognize amino acid sequence and bind a protein epitope" , *Proc Natl. Acad. Sci. USA.*, 93:7475-80 ). After ten cycles of selection, an RNA able to bind the protein Rev was obtained. However, the protein naturally binds RNA, and therefore, it was hard to know if the result could be generalized. In addition, the specificity was not convincingly proved. Gel-shift assays were used to demonstrate specificity, but using few other pure proteins and not a mixture of proteins (such as a nuclear extract, cytosol, etc.) as is normally done with gel-shifts. In addition, Western blots were not performed. Therefore, the procedure utilized did not prove the specificity in a convincingly way. It is important to note here that this is a very serious limitation, common to any work related to aptamers or SELEX: the specificity of the selected reagents for proteins have not been clearly proved with appropriated methods for antibodies such as Western blots, prepared using mixtures of total proteins, or immunohistochemistry or immunoprecipitations.

Accordingly, the prior art methods based on high protein-oligonucleotide affinity have not yielded reagents equivalent to antibodies, against any target protein native or denatured, nor has the specificity of such aptamers been convincingly proved. In addition, the procedures described so far are time-consuming and cumbersome, since they generally require between ten and twenty-five cycles of selection/amplification, as well as requiring separations of the sense and antisense strands during the selection procedures. Moreover, to obtain these reagents following the SELEX procedure, it is necessary to have the target protein in a pure and native form and the final product recognize only native proteins (Weiss et al. (1997) "RNA aptamers specifically interact with the prion protein PrP" , *J. Virol.,* 71:8790-7 ), which constitute another serious limitation. All in all, the efficiencies of the SELEX method are amply evidenced by the fact that even after ten years, reagents obtained by the SELEX procedure have not invaded, not even explored, the very demanding antibody market.

The difficulties of the prior art are overcome by the present invention, which combines the utilization of synthetic peptides, synthetic oligonucleotides, PCR, and most importantly, a strategy denominated "target switching", which implies a selection based mainly in specificity rather than in affinity. The reagents produced in this way are referred to as "oligobodies" because of their oligonucleotide nature and their behavior as antibodies. One of the advantages is that this invention generally requires no more than four or five cycles of selection/amplification. In addition, the reagents obtained have demonstrated very high specificity, which has been verified for the first time by using Western blots, immunohistochemistry and immunoprecipitations. Separation of the DNA strands is not required after amplification and, since a synthetic peptide is used, it is not necessary to possess the ultimate target in a pure form. In addition, it is possible to produce "monoclonal oligobodies" or "polyclonal oligobodies" against different regions of a protein without a need for "antigenic" regions, since the method is independent of the immune system. The fact that the oligobodies of the invention are not limited to "antigenic" regions of the target protein represents an important advantage over conventional antibodies. It is only necessary to know a portion of the protein (or ADN or ARN) sequence to be able to synthesize the corresponding peptide and to prepare oligobodies against the target protein. The oligobodies can be labeled with radioisotopes or with any conventional method to label oligonucleotides such as attachment of biotin.

The present invention describes the "oligobodies" and includes methods to efficiently obtain them from a combinatorial library. Oligobodies have properties similar to antibodies with respect to their specificity and applications. The procedure to obtain oligobodies described in the present invention does not require pure proteins. Moreover, this procedure also is applicable to situations in which the protein of interest has never been purified, since it is enough to know, or simply deduce, a partial coding sequence from the corresponding DNA or RNA sequences obtained from a database, and to have a very crude preparation of a tissue expressing the protein.

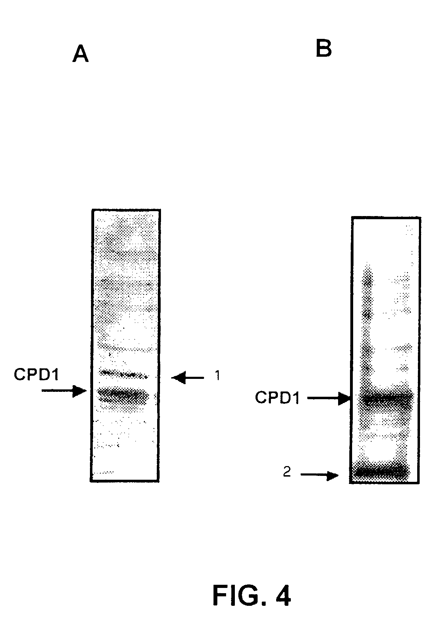

The strategy of the present invention is to use a synthetic peptide corresponding to a fragment of the target protein as a "temporary target", and to perform an initial selection of oligonucleotides able to recognize the synthetic peptide. Then, after a detectable binding is obtained, which usually occurs after as few as one or two cycles of selection/PCR-amplification, the synthetic peptide (temporary target) is replaced with the "final target", which preferably should be present in a very crude preparation of proteins. This strategy is very important to obtain oligobodies, which are specific for the target protein as is referred to as "target switching". If necessary, new cycles of selection are repeated with the final target until no further improvement in specificity is obtained, which usually occurs after as few as two or three selection cycles. In this way oligobodies (i.e., reagents that behave very similar to antibodies) are obtained by using synthetic peptides and a protein mixture including the ultimate target protein. In addition. according to the present invention, it is possible to prepare an "oligobody" against a protein deducted from a fragment of its ADN sequence, as shown in Figure 4 and Example 4 for CPD1. The protein CPD1 was never been purified in a laboratory and therefore is a good model system (GenBank Accession Number U89345).

In the procedure of the present invention the initial selection of a library subset by using the synthetic peptides as "temporary targets" and the subsequent "target switching" are highly important. The subset selected by using synthetic peptides is able to recognize, after switching targets, the "final target" in native, denatured or both forms. This is facilitated by the capacity of synthetic peptides to acquire multiple conformations in solution or solid phase (Kuroda et al. (1994) "Conformation of the C-terminus of endothelin-1 in aqueous solution studied by Monte-Carlo simulation" , *FEBS Lett.,* 355:263-6 ; Li et al. (1995) "Manipulation of peptide conformations by fine-tuning of the environment and/or the primary sequence" , *Biopolymers,* 35:667-75 ), some of which have the conformation(s) present in the native or denatured target protein. The specificity is obtained with this new subset, after switching targets, due to the presence of the final target together with a mixture of thousands of proteins (in Western blots, gel-shifts, etc.).

The present invention introduces an important innovation in the production of reagents to recognize proteins, allowing for the first time the recognition of both native and denatured proteins. In addition, the specificity of the oligobodies of the invention is clearly proved for the first time, by using methods that before were precluded to synthetic reagents and restricted only to antibodies, such as Western blots, immunohistochemistry and immunoprecipitations.

The final target has to be present in native or denatured form, depending on the need to produce oligobodies able to recognize one or the other form. Alternatively, it can be constituted by a mixture of both forms, when reagents capable of recognizing either form are to be developed. The use of synthetic peptides is also important. It avoids the need for a pure protein and also increases the probability of successfully obtain a reagent, since the conformational space of the target is not restricted, as in SELEX, to the few exposed and adequate conformations allowed in a native protein. Of course, if the sequence of the synthetic peptide is occluded in the native protein, it is probable that the oligobodies will recognize only the denatured protein and not the native protein. For these reasons it is recommended to use some computer program able to identify or predict regions in the target protein that would be exposed to the surface, and to use the corresponding synthetic peptides as "temporary targets".

On the other hand, there are additional advantages to the present procedure. For example, antigenic regions in peptides are not required so that the strategy is independent of the immune system. Instead, oligobodies can be obtained against specific regions in the target protein, or against consensus sequences if a family of proteins are to be identified, or against unique sequences, etc.

In addition, in accordance with one modification of the invention, specific binding regions can be obtained without any need to separate strands even temporarily in each cycle. The strategy for this involved performing an initial exponential amplification of the products extracted after binding, followed of a lineal amplification (or asymmetric PCR, using only one primer) in the presence of labeled nucleotides. By using this procedure, it is possible to obtain only one labeled strand, avoiding the cumbersome procedure of separating the sense and antisense strands.

Alternatively, the strands can be separated temporarily by rapid heating and cooling. However, sometimes a high disassociation temperature (Tm) in the selected oligonucleotides precludes the use of this simple procedure.

The invention also includes another, very important modification. The formation of species of high molecular weight after the initial amplification/selection cycles was observed. These species grow in size in each cycle and finally preclude any possible selection. This problem has been overcome by purifying the products obtained in each cycle, after the exponential amplification, by using separation in agarose gels. Then, in the following PCR amplification further polymer formation is not observed, although the molecular weight sometimes increases one or two times the initial library size due to reasons yet to be determined. However, these species, one or two fold higher in size, are perfectly viable for binding.

An alternative strategy to avoid the formation of undesired high molecular weight polymers is to increase the size of the forward and reverse primers used in the PCR amplification steps (in the initial library it is not necessary). Use of a 32-mer (forward) primer and a 38-mer (reverse) primer proved successful in avoiding high molecular weight polymers. Another alternative is to use a derivatized oligonucleotide in the sequence to avoid endo/exonuclease activity, which for reasons yet to be determined, this strategy inhibit the formation of undesired polymers during PCR amplification (results not shown).

Among the different strategies mentioned, a most important one, which is a key to the success of this invention, was to use a selection based on high specificity rather than high affinity. The procedures used in the past by other workers consist mainly in performing many cycles of selection searching for a subset of very high affinity, assuming that specificity will be an automatic consequence of the high affinity (See (Eaton et al. (1995) "Let's get specific: the relationship between specificity and affinity" , *Chem. Biol.,* 2:633-8 )). This assumption of the prior art, however, is not correct. For example, since aptamers can recognize small molecules, one could isolate an oligonucleotide recognizing with very high affinity the amino acid Arginine in a peptide strand. However, there is not doubt that this oligonucleotide would recognize the majority of proteins that have Arginines exposed to the surface, and almost all denatured proteins in a mixture. Therefore, although this aptamer would have high affinity for Arginine, it will also be highly non-specific. The present invention, on the other hand, makes use of the fact that an oligonucleotide binding a protein with intermediate affinity can still be highly specific. Indeed, this occurs with the majority of the monoclonal antibodies, which have only medium affinity but high specificity. For these reasons, the main strategy used in the present invention is to perform as soon as possible a selection looking for highly specific species without paying much attention to affinity in the initial steps. Consequently, only a minimal number of cycles sufficient to have a detectable signal are performed with the temporary target, and then the selection target is promptly switched to the final target present in a mixture of proteins in order to select mainly for specificity and not for affinity. Only after obtaining specific binding is attention paid to affinity, selecting those clones having sufficient affinity for detection purposes from among those that are highly specific. Thus, the strategy of the present invention is just the opposite to that utilized by SELEX, or similar procedures, which look for high affinity species, performing many cycles of selection/amplification. Once such a selection is done, the most important feature of a library (and also of nature), which is the diversity, has been minimized or lost. Then, it is too late to look for specific reagents among the subset of those exhibiting high affinity. The inventive strategy of selection for specificity rather than affinity has been very important since allowed us to make synthetic (oligonucleotide-based) antibodies or "oligobodies" for the first time, overcoming difficulties that has been very challenging for others during more than 10 years.

For the purposes of the present invention an oligobody or oligonucleotide may be considered to be "specifically bound" to a target molecule when said oligobody or oligonucleotide substantially recognizes only the target molecule, when the target molecule is present in a mixture with other molecules.

Another difficulty, which is inherent to the SELEX procedure, is that the high affinity compounds obtained are necessarily very restricted conformationally. so that any minor change in the conformation of the target would destroy the binding. It is probably for this reason that the SELEX procedure has been useful only for native proteins or small, rigid molecules.
1. In summary, the advantages of the present invention include the following: (1) Very few cycles of selection/amplification are required: usually two with the synthetic peptide and two or three with the mixture of proteins. Thus. maximum of four or five cycles are required compared to from ten to twenty-five normally required by prior art procedures. (2) The target protein does not need to be pure. On the contrary, it preferably should be used as a very crude preparation. This property enables oligobodies to be made against proteins with sequences deduced from databases, even if the target protein has never been purified. It is also possible to use this method to determine the correct reading frame by synthesizing oligobodies against the three possible frames and then determining which one really exists in nature. (3) After each amplification step, it is not necessary to split the strands permanently. It is only necessary to effect a temporary separation by heating/cooling or changing pH and neutralizing, or alternatively, to perform a lineal amplification to obtain one of the strands in excess. This is true during selection as well as for the final product. However, the dilution after heating the sample should be carefully tested to avoid re-association and the Tm has to be adequate. If the Tm is too high, then a different method to separate strands is required. (4) Compared to monoclonal antibodies, "oligobodies" have the advantage of rapid design and production, low cost, high affinity (nM range), independence of the immune system (it is not required that the target be antigenic) and better diffusibility (they are at least 5-fold smaller than natural antibodies). This last property improves the probability of using them successfully in the treatment of tumors and other diseases where the monoclonal antibodies have failed due to their limited accessibility to target. The oligobodies can be produced easily against different region of a target protein, independently of their antigenicity. (5) Chips similar to those used for DNA can be easily produced for use in diagnosis, gene expression studies, etc.

Accordingly, the present invention relates to oligobodies, to the procedures for obtaining them, and to the use of oligobodies in applications where antibodies have heretofore been used.

The present invention now will be described in more details with reference to the attached drawings in which:

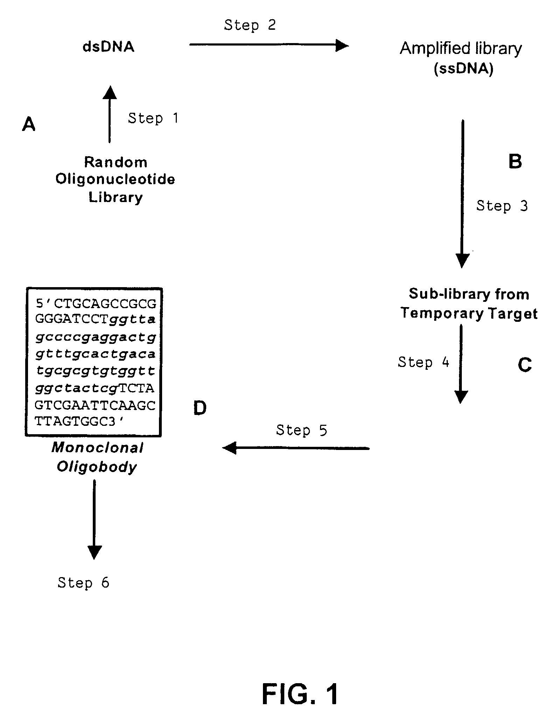

Figure 1 shows a scheme of the procedure used to obtain oligobodies. Step 1 is a PCR reaction. Step 2 involves separation into single-strands at 95°C for 10 minutes. Step 3 involves binding to a "temporary target" (synthetic peptide) for 8 hours at room temperature. This is followed by washing and elution at 95°C for 10 minutes in a PCR buffer. Then PCR is performed followed by separation into single-strands at 95° for 10 minutes. Step 4 involves "target switching" or, in other words, binding to a target protein present in a mixture for 6 hours at room temperature. Step 5 involves washing and elution followed by PCR of "polyclonal antibodies". This is followed by cloning, screening and sequencing. Step 6 could involve Western blot, histology, or immunoprecipitation etc.

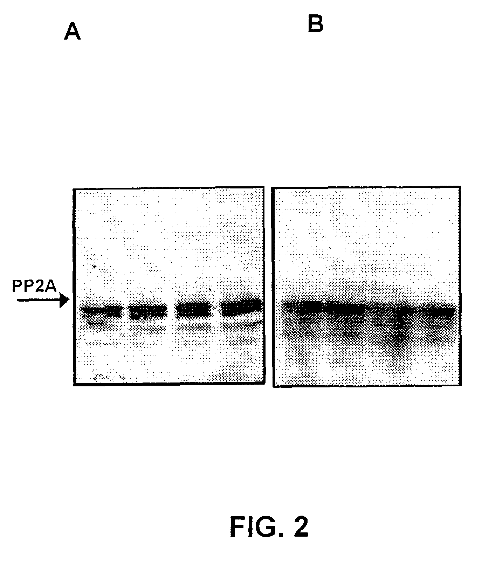

Figure 2 shows a Western blot of total proteins from cerebellum stained with polyclonal antibodies specific for the protein PP2A in which "A" identifies a Western blot obtained with a commercial polyclonal antibody developed with a secondary antibody (rabbit antibody) coupled to alkaline-phosphatase (control), and "B" identifies a "Western blot" obtained by using the "oligobody" labelled with ³²P and developed by autoradiography.

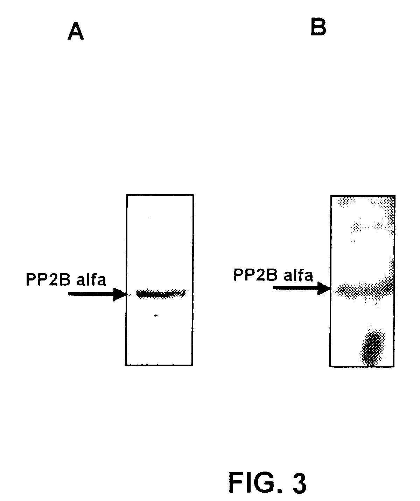

Figure 3 shows a Western blot of total proteins from cerebellum developed with polyclonal antibodies against the protein PP2Bα in which "A" identifies a Western blot obtained using a commercial polyclonal antibody against PP2Bα and developed with a secondary antibody (rabbit antibody) coupled to alkaline-phosphatase (control), and "B" identifies a "Western blot" obtained by using a polyclonal "oligobody" labelled with ³²P and developed by autoradiography.

Figure 4 shows a Western blot of total proteins from cerebellum developed by using a polyclonal antibodies against the protein CPD1 in which "A" identifies a Western blot obtained by using a polyclonal antibody (rabbit antibody) produced in rabbits by injection of a synthetic peptide corresponding to CPD1 and developed with a secondary antibody coupled to alkaline-phosphatase (control), and "B" identifies a "Western blot" obtained by using a polyclonal "oligobody" obtained with the same synthetic peptide, labelled with ³²P and developed by autoradiography. Both antibodies recognize multiple isoforms of CPD1. Reference numeral 1 refers to a non specific band corresponding to pre-immune serum. Reference numeral 2 refers to the gel front.

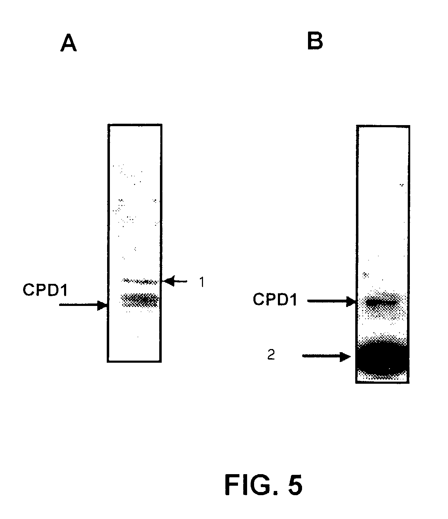

Figure 5 shows a Western blot of total proteins from cerebellum developed by using antibodies against CPD1 in which "A" identifies a Western blot obtained by using a polyclonal antibody made in rabbits utilizing the synthetic peptide corresponding to CPD1 and developed with a secondary antibody coupled to alkaline-phosphatase (control), and "B" identifies a Western blot obtained by using a monoclonal "oligobody" labeled with ³²P and developed by autoradiography. Reference numeral 1 refers to a non specific band corresponding to pre-immune serum. Reference numeral 2 refers to the gel front.

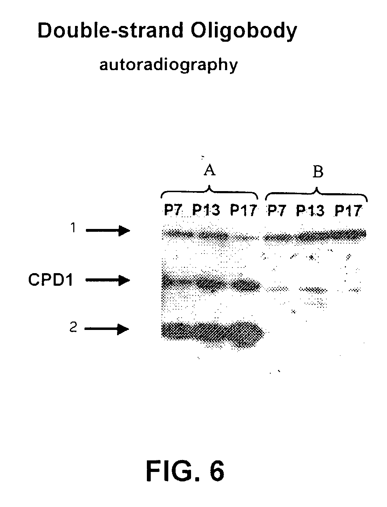

Figure 6 shows a Western blot of total proteins from cerebellum of different postnatal days (P7, P13, and P17) developed using a ³²P-labeled double-stranded monoclonal "oligobody" against the protein CPD1. The strands were separated by heating the oligobody solution to 95°C just before use, and the blot was developed by autoradiography. The same isoforms detected using a rabbit polyclonal antibody (see Fig. 5) are observed. Reference numeral 1 refers to a HMW-CPD1 isoform. Reference numeral 2 refers to the gel front. Columns marked A relate to a homogenate. Columns marked B relate to a supernatant 16,000 x g-10'.

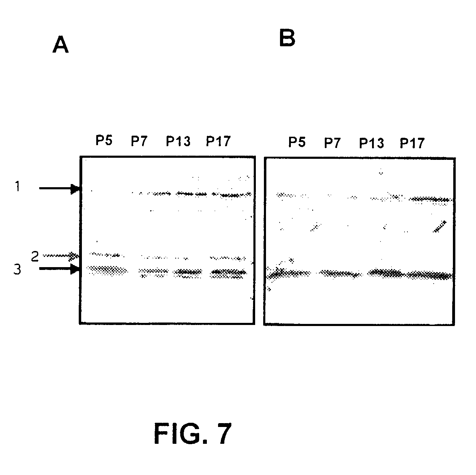

Figure 7 shows a Western blot of total proteins from cerebellum of different postnatal days (P5, P7, P13, and P17), developed using antibodies against the protein CPD1 in which "A " identifies a Western blot obtained using a rabbit polyclonal antibody made against the CPD1 peptide and developed with a secondary antibody coupled to alkaline-phosphatase (control), and "B" identifies a Western blot obtained with a double stranded monoclonal "oligobody" biotynilated during PCR amplification and developed by using avidin-alkaline-phosphatase. The same isoforms are observed with both antibodies. Reference numeral 1 refers to a high molecular weight CPD1 band. Reference numeral 2 refers to a preimmune band. Reference numeral 3 refers to a low molecular weight CPD1 band.

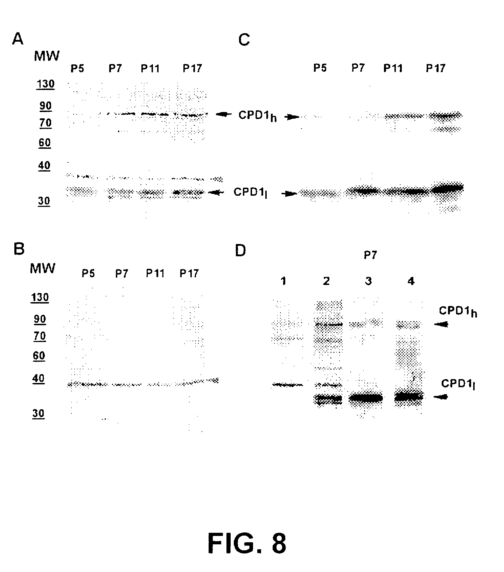

Figure 8 shows "Western blots" and "immunoprecipitations" using a monoclonal oligobody against CPD1 in which freshly isolated cerebella from mice at postnatal ages from 5 to 17 days (P5, P7, P11 and P17) were homogenized, and 100 micrograms of protein run in a 10% SDS-PAGE, transferred to nitrocellulose membrane, and blocked with PBS-BSA 5%. In the figure, "A" identifies a Western blot developed by incubation with rabbit polyclonal primary antibody performed overnight at 4° C followed by the secondary antibody for 1 hour at room temperature, and developed using goat anti-rabbit phosphatase alkaline conjugate and NBT/BCIP; "B" identifies the results obtained using the preimmune rabbit serum developed as in A: "C" identifies a Western blot developed using biotinylated monoclonal oligobodies, incubated 1 hour at room temperature in PBS-5%BSA. washed 3 times with PBS and developed with streptavidin-PA, and "D" identifies a comparison of Western blots at P7 and "Immunoprecipitation" of ³⁵S-CPD1 in which column "1" is a Western blot at postnatal day 7 using pre-immune rabbit serum; column "2" is a Western blot using rabbit polyclonal antibody; column "3" is a Western blot using monoclonal oligobody, and column "4" is an "Immunoprecipitation" using the monoclonal oligobody and proteins labeled with ³⁵S. The letters "h" and "l" stand for high and low molecular weight isoforms, and the gray arrows show a band corresponding to the preimmune serum.

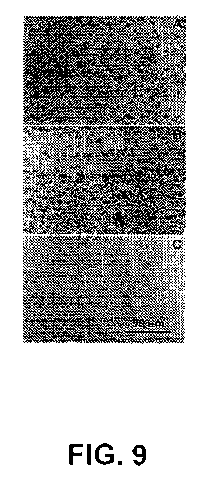

Figure 9 shows an Immunohistochemistry of CPD1 in which mouse cerebella (C57BL/6J mice) were dissected at postnatal day 7, fixed in alcohol/acid (95% Ethanol: 5% acetic acid, 4 hours at -20°C), dehydrated, and embedded in paraffin. Five µm tissue slices were mounted in silanized (Silane, Sigma Co., St. Louis, MA) coverglass, deparaffinized, rehydrated and blocked using PBS-5%BSA during 1 hour. In the figure, "A" identifies slices exposed to affinity-purified rabbit polyclonal antibodies overnight at 4 °C, rinsed twice with PBS, incubated with the secondary antibody (goat anti-rabbit peroxidase 1:1000) for one hour and developed with DAB (Gibco BRL, Gaithersburg, MD); "B" identifies an "Immunohistochemistry" staining obtained with a biotinylated-monoclonal oligobody. The reagent was made by adding a 5'-biotinilated primer to the PCR reaction (5'-Biot-CTGCAGCCGCGGGGATCCT-3'). After PCR amplification, the cDNA strands were separated by heat (94 °C, 5') and diluted ten times with PBS-5% BSA. After incubation of slides with PBS-5% BSA to block nonspecific sites, oligobodies were incubated with the tissue for 1 hour at RT and then developed with streptavidin-peroxidase (Promega Corporation, Madison, WI). The presence of biotin does not affect the PCR amplification or the binding to the target, since the bands obtained in a Western blot stained with biotinylated-oligobodies were identical to those developed using ³²P-labelled-oligobodies (results not shown); "C" identifies control samples made preincubating the oligobodies with an excess of blocking peptide (overnight at 4 °C) followed by incubation and development as indicated in "B".

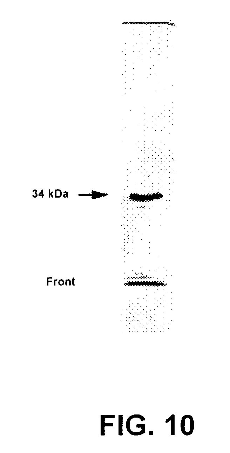

Figure 10 shows a Western blot obtained with a synthetic oligobody against CPD1 made in an oligonucleotide synthesizer using the oligobody sequence illustrated in Figure 1. This reagent constitutes the first synthetic antibody-like reagent made entirely by chemical synthesis, able to recognize a protein in a Western blot as it was a natural antibody.

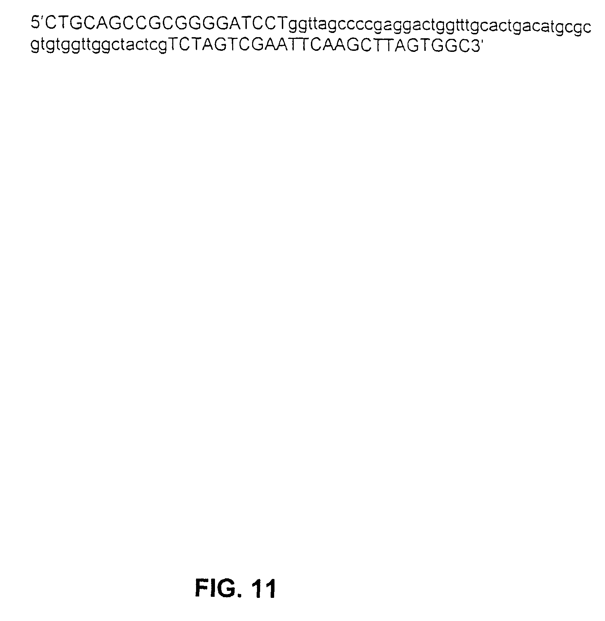

Figure 11 shows a sequence corresponding to one of the many possible oligobodies specific for CPD1 that is obtainable by the present method.

This invention describes a procedure to obtain "oligobodies", i.e., binding reagents which are obtained by using a library of oligonucleotides of random sequence, comprising molecules which have the unique ability to recognize proteins very specifically either in native or denatured form, and the unique ability to behave as antibodies in assays including, but not limited to, Western blots, immunohistochemistry and immuno- precipitations, which has been obtained by a procedure comprising the following steps:
i) Synthesising a library of oligonucleotides containing a random core sequence and exponentially amplifying this library by PCR using forward and reverse primers, a procedure that minimizes truncated forms that appear during synthesis; thereafter, purifying the amplified library and subjecting the purified products to a lineal or asymmetrical PCR amplification using only one primer;
ii) Incubating the lineally amplified product obtained in step i) with a "temporary target" made with a synthetic peptide corresponding to a region of the target protein. Preferably the synthetic peptide is bound to a solid matrix, but binding into liquid phase could be also used, followed by separation between bound and free species by any of several methods known in the art;
iii) Selecting oligonucleotides which specifically bind to the temporary target by washing and subsequent elution of oligonucleotides bound to the target;
iv) Thereafter exponentially amplifying the oligonucleotides selected in step iii) by PCR, purifiying the amplified product, and subsequently subjecting the purified product to lineal PCR amplification using only one primer in the presence of labeling agent. The labeling agent may be a radiolabeled or a biotinylated base, or a fluorescing agent such as a fluorescein derivative, or any other labeling agent heretofore used to label oligonucleotides;
v) Repeating steps ii), iii) and iv) until a detectable binding is obtained and recovering the selected oligonucleotides bound to the temporary target. Typically steps ii), iii) and iv) need only be repeated once or twice;
vi) Changing the synthetic peptide or "temporary target" by a mixture of proteins containing the "final target" (target switching), incubating the oligonucleotides recovered in step v with the final target, and selecting oligonucleotides which bind specifically to the final target.
vii) Exponentially amplifying the oligonucleotides selected in step vi) by PCR, purifying the amplified product, subsequently subjecting the purified product to lineal PCR amplification using only one primer, and recovering the lineally amplified oligonucleotides;
viii) Repeating steps vi) and vii) until further increases in binding specificity for the target molecule are no longer obtained, and collecting the resulting specifically bound oligonucleotides, which constitutes the "polyclonal oligobodies"; These specific binding oligonucleotides or polyclonal oligobodies can be PCR amplified, ligated into a suitable vector such as vector-T (Promega Corp.), cloned, screened and purified to obtain "monoclonal oligobodies" having a consistent defined sequence. The monoclonal oligobodies can then be obtained in useful quantities by PCR amplification, plasmid digestion, or entirely by chemical synthesis using an oligonucleotide synthesizer.

For the purposes of the present invention, "increased specificity" can be said to have been obtained from one step of selection to the other, when the number and/or intensity of non-targeting signals (for example, secondary spots seen in a Western blot) is reduced from one step of selection to the other, and the signal corresponding to the target molecule has been maintained or increased compared to the previous step.

The procedure to obtain oligobodies is summarized in Figure 1. To control the different steps of the procedure, the oligonucleotides are labeled using a ³²P-labeled nucleotide during the lineal PCR amplification steps. They could also be labeled with other procedures such as biotinylation without any problem. In this way, the polyclonal oligobodies obtained in the last step can be directly used for Western blots, dot-blots, immunohistochemistry and immunoprecipitation (the later using for example biotin labeling). Alternatively, at the end of the selection procedure, it is possible to label the oligobodies by using any other procedure normally used to labeled oligonucleotide probes such as using terminal transferase with bases that have been biotinylated, bound to digoxigenin or fluorescent compounds, hybridization with secondary probes, etc. Biotin labeling is particularly useful for immunoprecipitations, affinity columns, treatment of diseases, etc. Many other alternatives for labeling exist which are known in the art. In Figure 7, an example of a monoclonal oligobody labeled by adding a biotinylated primer (forward primer) during the last amplification step is shown.

Some fragment of the target protein must be known to design the synthetic peptide used as a temporary target. Preferably, although not necessarily, the open reading frame (ORF) will also be known. If the ORF is not known it will be necessary to synthesize peptides corresponding to the three reading frames and test each one separately. If the corresponding sequences are not available, then a pure protein will have to be used as temporary target.

If the objective is to develop oligobodies able to recognize the native protein and enough sequence information is available, it is convenient to use any software useful to predict antigenic sites to select the fragment used as the temporary target. In this way it is possible to increase the probability of the native protein being recognized by the oligobody. The reason why is that the predicted "antigenic regions" are generally at the surface of the molecule and not because they are "antigenic", since as noted above, antigenicity is not required because the method of the invention is independent of the immune system. One among many suitable computer programs for predicting antigenic regions is found in the GCG package from the Genetic Computer Group (Oxford Molecular, www.gcg.com). The temporary targets peptides are preferably synthetized using classic solid phase methods of modern peptide synthesizers.

### EXAMPLE 1

### EXPERIMENTAL PROCEDURES

The procedure to obtain oligobodies is summarized in Figure 1.

### Synthesis and amplification of the oligonucleotide library:

Generally, to obtain a PCR-amplified library three oligonucleotides are required. One is the forward primer, which contains a non-random sequence of about twenty to forty bases. The second is the reverse primer, which also contains a similar non-random sequence. The third is the combinatorial library, which contains the sequence corresponding to the forward primer followed by a random core sequence that could be variable in extension, normally between thirty and eighty bases, followed by another non-random sequence complementary to the reverse primer. The forward (or reverse) primer may optionally be linked to biotin to facilitate separation of strands or to label the oligonucleotides. The oligonucleotides used in this example were the following:
Oligo 1 (forward primer, region 5'): 5'-CTGCAGCCGCGGGGATCCT-3' (SEQ ID NO:1)
Oligo 2 (reverse primer, region 3'): 5-GCCACTAAGCTTGAATTCGACTAGA-3' (SEQ ID NO:2)
Oligo 3 (library): 5-CTGCAGCCGCGGGGATCCN₅₆TCTAGTCGAATTACAAGCTT AGTGGC-3 (SEQ ID NO:3),
where N₅₆ represents the random polynucleotide core library sequences. Of course, these oligonucleotides are only examples and could be modified in their extension or composition by using derivatized oligonucleotides and primer sequences without departing from the invention. Interestingly, it was observed that derivatized oligonucleotides, using for example biotinylation, avoid the formation of undesired high molecular weigh polymers, for reasons yet to be determined (results not shown). It is noteworthy that others have not reported the formation of these undesired high molecular weight polymers yet.

After synthesis, only the primers should be purified by HPLC. Since a 100-mer oligonucleotide (or longer) has been chosen, the yield during synthesis is low. The majority of the product is comprised of truncated by-products and a premature HPLC purification before PCR-amplification would yield only truncated forms. To eliminate the impurities it is necessary to amplify the library by using PCR with the corresponding primers (oligo1 and oligo2). By using this amplification step followed by purification in agarose gels, the final library is obtained.

### Library amplification and labeling:

To amplify the original library we used the following protocol:

The following protocol was used to amplify the original library. The original lyophilized oligonucleotides were dissolved in water free of DNAses to a final concentration of 100 µM.
Primer, oligo1: 4.8 µl of a stock solution of 25 µM (final concentration 1.2 µM)
Primer, oligo2: 4.8 µl of a stock solution of 25 µM (final concentration 1.2 µM)
Taq polymerase: 1 µl of a stock solution of 5 U/µl (final concentration 0.05 U/µl)
Template, oligo 3: 10 µl of a stock solution of 10 µM (final concentration 5 µM).
Buffer: 10 µl of Buffer 10X (KCI 500 mM, Tris-HCI 100 mM pH 9.0 at 25 °C, Triton X-100 1%.)
MgCl₂: 6 µl of a stock solution of 25 mM (final concentration 1.5 mM).
Water free of DNAses, 63.5 µl (final volume 100 µl).
Bases (DNTPs): 1.0 µl of a stock solution of 25 mM (final concentration 0.25 mM of each DNTP).
Label: 1.5 x 10⁶ cpm ((α-³²P) dCTP, 3000 Ci/mmol, NEG 513H, NEN).

The amplification was carried out using cycles programmed as follows: Initial cycle: 5' at 94°C
35 cycles of: 94°C for 30". 50 °C for 30", 72°C for 60"
Final extension: 5' at 72°C.

These parameters can be optimized according to the extension of the original library (i.e. final extension omitted, etc.).

In this way, double strands containing random sequences were obtained. These chains were separated permanently in the initial assays as described by others. It was subsequently discovered that permanent separation of the strands in general is not necessary, therefore, often only a temporary separation was used (see Figures 5 and 6). Finally, it was observed that when a lineal (asymmetrical) amplification was used, a temporary separation of strands was not required because the PCR product has only one strand in this case. Therefore. this was the preferred method to use for amplifications.

Initially, to separate strands the method described by Bock et al. (Bock et al. (1992) "Selection of single-stranded DNA molecules that bind and inhibit human thrombin," *Nature,* 355:564-6), was followed with some modifications. After PCR amplification and labeling in the presence of the biotinylated primer 2, the two strands were separated by using streptavidin MagneSphere paramagnetic beads (Promega Corp.). The double strands bind to the avidin through the end containing the biotinylated primer. The other strand is then eluted by using NaOH 0.15 N. The procedure in detail is as follows:

Eighty-five µl of PCR amplification product were mixed with 85 µl of H₂O (distilled and sterilized by autoclave) and 85 µl of streptavidin MagneSphere paramagnetic particles. The slurry was incubated during 10 minutes at room temperature. Then, the beads were separated from the solution using a magnetic rack (Promega Corp.) and the supernatant was discarded. The beads were washed for times with 220 µl portions of 0.1% SSC. Finally, the beads were resuspended in 73 µl of 0.15 N NaOH and separated again after one minute and the supernatant recovered. The extraction with NAOH 0.15 N was repeated once, and the supernatants were neutralized by adding 46 µl of 0.2M NaAc. In this way. single strand oligonucleotides were obtained, which were then incubated with the nitrocellulose membranes initially containing the "temporary target" and later the "final target".

Although the selection of the oligonucleotides according to this invention has been described using streptavidin MagneSphere paramagnetic beads, it should be understood that other methods to separate both strands are also available. For example, the cDNA can be inserted in a plasmid and then translated to RNA, retro-transcribed to cDNA followed by treatment with RNAase to liberate single strand cDNA. Other alternative procedures are also possible and very well known in the art. However, it has been found that permanent separation of both strands is often unnecessary, which highly simplify the procedure. In the majority of the cases only a temporary separation of strands according to the following procedure is required:

### Temporary separation of DNA strands:

As described above, it has been observed that after initial PCR amplification a permanent separation of strands is not necessary. In this way, the procedure was greatly simplified. This also has additional advantages; since both strands are present, the number of members of the library present is double and the possibility of obtaining a useful compound is increased. In addition, the reagents can be stored as double strands and separated just before use, which significantly increases their stability. Permanent separation of strands may be required in exceptional cases; for example, when high affinity species able to bind to the target are not present and competence with the antisense strand is then significant, or when the Tm of the selected oligonucleotides is very high. The preferred method for separating the strands is very simple: heating the PCR product for 5 minutes at 90° C, followed by rapid cooling over ice. There are other possible methods; for example, adding 0.1 N NaOH followed by rapid neutralization.

### Lineal (or asymmetrical amplification):

As noted, it has been found that even temporary separation of the strands is unnecessary if a lineal (asymmetrical) amplification is performed after the initial exponential PCR in each selection cycle. In other words, after initial PCR amplification without radioactive labeling, the product is run and purified from an agarose gel to eliminate primers and possible polymeric byproducts, and finally an asymmetrical PCR is performed with only one primer. This PCR is performed under the same conditions described above for exponential (normal) PCR. although it is done in the presence of only one primer and in the presence of radiolabeling. Preferably the primer used is the forward primer, i.e., the primer corresponding to the 5'region. The region of the gel containing the cDNA band is visualized using Ethidium Bromide, and the purification from the gels is done by ctting-out the visualized region, followed by elution using centrifucation at 12.000 x g for 10 minutes, over a layer of cellulose prepared using Whatman 1 paper (see Chuang and Blattner (1994) "Ultrafast DNA recovery from agarose by centrifugation through a paper slurry" , *Biotechniques*, 17:634, 636 )). GenClean is not convenient due to its low yield for short cDNA fragments.

### Binding of the synthetic peptide to a solid matrix:

In order to isolate the members of the library that bind to the "temporary target", i.e., the synthetic peptide, a method of separation is required. Although in principle solid or liquid phase methods could be used, it is preferred to use a solid phase method due to its simplicity. Among the different solid phase methods, adsorption over nitrocellulose was selected for the following experiments. A approximately 0.25 m² fragment of nitrocellulose was added to a solution containing the temporary target peptide at a saturating concentration. After equilibration for about two hours, the membrane was allowed to dry in air. The free peptide was washed three times for five minutes each time using PBS (pH 7.4).

In some cases it was necessary to have the synthetic peptide cross-linked to BSA to be able to perform the initial selection, or latter the target switching (Examples 2 and 3 were made with synthetic peptides cross-linked to PP2A and PP2B). A failure in the initial selection often is indicative of a poor peptide binding to nitrocellulose, which is facilitated by cross-linking to BSA. A failure during target switching using uncross-linked peptides suggest that perhaps the cross-linking procedure helps to fix the peptide in a conformation mimicking the actual conformation in the protein chain. However, the exact reasons for the cross-linking requirement remain to be determined. The method to use for cross-linking depends on the peptide sequence. There are several possibilities very well know in the art, often applied to produce antibodies in rabbits.

### Selection of the oligobodies:

The first step is a pre-adsorption of the oligonucleotides from the library that bind to the solid matrix or to the protein used to block the non-specific sites in the matrix (nitrocellulose, activated Sepharose, etc.) used to contain the temporary target (synthetic peptide) or the final target (the protein). In the following experiments, nitrocellulose was used as a matrix, but any other solid-phase or liquid phase method for separating bound and free species could be used. The nitrocellulose (without peptide, 0.25 cm²) was first blocked using casein 5% (dry, non-fat milk) for 30 minutes. Then, 70 µl of the PCR-amplified/labeled oligonucleotide library were added to 200 µl of a solution containing casein (50 mg/ml in PBS buffer) together with the nitrocellulose matrix and incubated over night to perform the pre-adsorption. Once the pre-adsorption was completed, the remaining oligonucleotides were incubated overnight with the matrix containing the target peptide (temporary target) and then the steps i) to viii) as described above were followed.

In some cases this pre-adsorption step was not necessary. It is sufficient to carry out one or two selection steps with the peptide bound to nitrocellulose with the peptide present in a saturating concentration, and then to perform a dot-blot with the target protein if the target is a native protein, or a Western blot with the target protein if the target is a denatured protein, (now with the nitrocellulose blocked with casein) and then clone the resulting products as described below. It is very important to use an appropriate selection method when switching from the temporary target to the final target. If the objective is to isolate oligobodies able to recognize a native protein, then dot blots, separation in liquid phase, immunohistochemistry, gel shifts, column separation or other non-denaturing methods, etc. should be used in the selection. In other words, a separation method should be selected that will keep the target protein as close as possible to its native state. This is because oligobodies against native proteins might be very sensitive to conformational changes. On the other hand, if the objective is to isolate oligobodies able to recognize denatured target proteins, then methods that maintain the targets in this way should be used, for example, Western blots. Sometimes, however, as occurs with natural antibodies, an oligobody made against a denatured target can also recognize the native protein and vice-versa. Indeed, this occurs in the following example with CPD1, but this is not always the case.

### Cloning:

The "oligobodies" obtained following the procedure described above are polyclonal because they are originated from a library of random conformers. However, the different members of the library selected by the procedure set forth above also can be obtained in monoclonal form. To obtain monoclonal "oligobodies", after PCR amplification of the final polyclonal oligobodies using normal, non-biotinylated primers, the oligonucleotides are ligated into a suitable vector such as vector-T (Promega Corp.) or some other vector and transfected into competent bacteria following the directions of the manufacturer. After transfection, several clones are isolated, and the insert amplified using the "colony-PCR" method. Then, the capacity of each insert to bind the target protein is analyzed using Western blots. In this way, the monoclonal oligobody of Example 5 was obtained, which was then utilized as a starting material in Examples 6 to 10.

### Characterization of the oligobodies to determine their specificity:

Western blots (Fig. 2-8) made from total proteins isolated from cerebellum were utilized to determine the specificity of the oligobodies. The results were also confirmed by using immunoprecipitation (example 8 and Fig. 8) and immunohistochemistry (example 9 and Fig. 9).

Chemical synthesized oligobodies: once a monoclonal oligobody is selected, cloned and sequenced, its sequence can be used to construct an entirely chemical synthesized oligobody by using an oligonucleotide synthesizer. A Western blot developed using such a reagent is shown in Figure 10. This constitutes the first chemical synthesized reagent able to perform like an antibody in a Western blot.

### Usefulness of the obtained reagents:

The resulting oligobodies are useful for all the applications in which monoclonal or polyclonal antibodies are used. Moreover, as shown in the Fig. 7, it is possible to label the oligobodies utilizing the primer1 derivatized with biotin, as is normally done with monoclonal or polyclonal antibodies. The derivatization does not affect the results. See Example 7 below for more details. The oligobodies are also useful for immunoprecipitations, as shown in Fig. 8, which implies that in some cases the oligobodies can recognize both native and denatured proteins since the protein was maintained in native form during the precipitation procedure (see Example 8). In addition, as shown in Fig. 9, the oligobodies were also useful as reagents for immunohistochemistry (See Example 9).

These results demonstrate that the oligobodies can replace natural monoclonal or polyclonal antibodies in applications such as diagnosis tests, immunohistochemistry, immunoprecipitations. etc. Labeling with biotin is only an example. It is also possible to label the oligobodies by using fluorescent compounds, to couple them to other proteins as carriers, and/or to use them as carriers for therapeutic drugs, etc. It is thus apparent that the oligobodies of the present invention can replace monoclonal or polyclonal antibodies in almost any application, with important advantages over natural antibodies as described above. After being cloned and sequenced, these oligonucleotides can easily be synthesized on a chip or other solid matrix to be used in diagnosis or other similar applications.

### Example 2

### Production of Polyclonal Oligobodies against PP2A:

A library of single-strand DNA molecules was made with synthetic oligonucleotides that contained a core random region flanked by fixed, know sequences. The library was amplified by PCR utilizing primers corresponding to the 5'and 3' ends. The reverse primer was biotinylated. The strands were then separated using binding to streptavidin-beads, elution with NaOH and neutralization. The oligonucleotide mixture was incubated with the synthetic peptide, as temporary target, which was bound to nitrocellulose. The synthetic peptide corresponded to a fragment of the target protein. In this case, the peptide corresponded to residues 298-309 (Pro-His-Val-Thr-Arg-Arg-Thr-Pro-Asp-Tyr-Phe-Leu) (SEQ ID NO:4) of the catalytic subunit C of the protein phosphatase 2A (PP2A) and it was cross-linked to BSA. Oligonucleotides which bound to the peptide were selected and amplified by PCR using radiolabeling. The double strands were separated using the method of "permanent" separation of strands, and the resulting oligonucleotides incubated again with the peptide bound to nitrocellulose. The amount (in cpm) of bound oligonucleotide was then determined by dot-blot quantification. The cycle was repeated until an appreciable binding was detected compared to controls. In this case three cycles were required. The results are summarized in Table 1 and represent an example of quantification of the amount of oligonucleotide bound to the synthetic peptide, used as temporary target during initial cycles of selection/amplification. The numerical values in the second and third columns of Table 1 represent the cpm bound to the synthetic peptide and the corresponding controls during the first three consecutive cycles, measured utilizing a dot-blot assay.

**Table 1**

| Selection Cycle | Peptide Bound CPM | Control Bound CPM | Ratio Peptide/Control |
|---|---|---|---|
| First cycle | 460 | 600 | 0.76 |
| Second cycle | 6005 | 880 | 6.8 |
| Third cycle | 9157 | 258 | 35.5 |

The "target switching" step was followed. In this step, the mixture of oligonucleotides selected in the previous step was used to develop a Western blot, as if the oligonucleotide mixture were a normal radiolabeled antibody. At the same time, the spot corresponding to the PP2A was identified by using commercial antibodies. An additional selection was done using PCR-amplification of the oligonucleotides bound to this spot. The result obtained in a new Western blot was compared to the one obtained by using the commercial available rabbit antibody. As can be seen from Fig. 1, the results are identical.

### Example 3: Production of Polyclonal Oligobodies against PP2B, catalytic subunit (subunit α).

Following the same procedure described in Example 2, polyclonal oligobodies were obtained against the subunit α of the protein phosphatase 2B (PP2B). The synthetic peptide used to prepare the oligobody was the same commercially available to block the commercial antibody made in rabbits, and corresponded to amino acids 268-280 (Arg-Met-Pro-Pro-Arg-Arg-Asp-Ala-Met-Pro-Ser-Asp-Ala) (SEQ ID NO: 5). It was also cross-linked to BSA. The results obtained with the polyclonal oligobody (developed by autoradiography), compared with those obtained with the commercial antibody (developed with a second antibody containing alkaline phosphatase), were identical as can be seen from Fig. 3.

### Example 4: Production of Polyclonal oligobodies against CPD1 (a protein deducted from its cDNA sequence, GenBank MMU89345).

Using a synthetic peptide, not cross-linked to BSA, corresponding to a fragment of CPD1 (Cys-Pro-Asn-Leu-Thr-Tyr-Leu-Asp-Leu-Ser-Gly-Asn-Lys-lle-Lys) (SEQ ID NO:6) the procedure described in Example 2 was followed to obtain a polyclonal oligobody. The resulting oligobody behaved exactly as the natural polyclonal antibody obtained in rabbit by using the same peptide cross-linked to BSA using glutaraldehyde. The results are shown in Fig. 4.

### Example 5: Production of Monoclonal Oligobodies against CPD1.

The polyclonal oligobodies obtained in Example 4 were PCR-amplified, ligated into vector-T (Promega Corp.) and cloned. Several clones were isolated, and their inserts were tested using mini-Western blots for their capacity to bind CPD1. One clone was selected, PCR-amplified, the strands separated and tested for antibody-like activity. As shown in Figure 5, this oligobody, which was now monoclonal, behaved identical to the polyclonal oligobodies obtained in Example 4.

The monoclonal oligobody was sequenced and found to have the following sequence: 5'-CTGCAGCCGCGGGGATCCTggttagccccgaggactggtttgcactgacatgcgcgtgt ggttggctactcgTCTAGTCGAATTCAAGCTTAGTGGC-3' (SEQ ID NO:7). This oligonucleotide is only one example of many possible oligobodies specific for the protein CPD1.

### Example 6: Production of double-stranded Monoclonal Oligobodies against CPD1.

The double strands of the oligobody obtained in Example 5 were temporarily separated before incubation by heating for 5 minutes at 90°C, followed by rapid cooling over ice. Alternatively, this temporary separation could be performed in several ways; for example, by changing the pH with NaOH followed by rapid neutralization with a buffer. After cooling, the oligonucleotide was used to develop the Western blot shown in Fig. 6. As can be seen from Fig. 6, the double-stranded, monoclonal oligobody obtained after PCR amplification, used without a permanent separation of strands, was as effective as the one used after permanent separation of both strands (compare Figs. 4 and 5). This example demonstrates that separation of the double strands is not necessary during the selection process or during binding reactions of the final product.

### Example 7: Labeling of a Monoclonal Oligobody with biotin.

The oligobody obtained in Example 5 was labeled using biotin. The labeling was done by using a biotinylated forward primer during the last PCR-amplification. As can be seen from Fig. 7, the biotinilization did not affect the binding properties of the oligobody, since the results were identical to those obtained before by radiolabeling. This example demonstrates that, in addition to radiolabeling, other methods typically used to label oligonucleotides or antibodies could be used to label the oligobodies of the present invention.

### Example 8: Western blots and immunoprecipitations with a monoclonal oligobody against CPD1.

Freshly isolated cerebella were homogenized in PBS containing a cocktail of proteinase inhibitors (final concentration: EDTA 10 mM, phenantroline 10 mM, E-64 10 µM, leupeptin 100 µM, aprotinin 10 mg/ml, pepstatin A 10 µM) (Sigma Co., St. Louis, MA). One hundred micrograms of protein were run in a 10% SDS-PAGE, transferred to nitrocellulose membrane, blocked with PBS-BSA 5% and developed using either rabbit polyclonal antibodies incubated ON at 4°C and developed with secondary antibody as described above, or biotynilated-monoclonal oligobodies incubated one hour at room temperature in PBS+5% BSA and developed with streptavidin-AP. For immunoprecipitations, the published procedure of Radrizzani and co-workers (Radrizzani et al. (1995) "Developmental regulation of Thy 1.2 rate of synthesis in the mouse cerebellum" , *J. Neurosci. Res.,* 42:220-7) was followed with a few modifications. Briefly, small fragments of mouse cerebellum where incubated in the presence of ³⁵S-methionine, the protein was extracted, and immunoprecipitations were performed using a monoclonal oligobody labeled which had been labeled with biotin by using PCR amplification in the presence of a biotinylated upstream primer, and streptavidin paramagnetic beads (Promega Corp.). As indicated before in previous examples, the insert corresponding to the CPD1 oligobody was PCR-amplified using a biotinylated primer. A Western blot was performed again to compare results with the immunoprecipitation of CPD1. As can be seen in Fig. 8, the monoclonal oligobody again behaves in Western blots exactly as its polyclonal counterpart developed in rabbits using the same synthetic peptide. The only difference observed is the presence of bands corresponding to the pre-immune serum when polyclonal antibodies were used. According to Northern blots, CPD1 is a protein expressed in cerebellum in two isoforms: CPD1ₕ and CPD1ₗ (results not shown). Western blots from cerebellum, developed with rabbit polyclonal antibodies or with monoclonal oligobodies, also show two isoforms, which are differentially expressed during development from postnatal day 5 to 17 (P5 to P17), with a 34 Kd isoform being more abundant. These results were obtained labeling the oligobodies with biotin after cloning, thereby reconfirming that biotin labeling can be carried out without affecting the ability of oligobodies to recognize the target. Similar results were obtained labeling the oligonucleotides with ³²P-dCTP during PCR amplification. This indicates that, at least in this case, the ends of the oligobodies corresponding to the forward and reverse primer sequences are probably not involved in binding. Interestingly, the oligobodies labeled with biotin were also useful to "immunoprecipitate" CPD1 (See Fig. 8 D). This indicates that this oligobody can recognize both the native and the denatured CPD1, as predicted by the GCG computer program. These results demonstrate that oligobodies can recognize both native (e.g. in immunoprecipitation) and denatured proteins (e.g., in Western blots) and constitutes the first example of an specific "immunoprecipitation" made with a synthetic antibody-like reagent.

### Example 9: "Immunohistochemistry" using a monoclonal oligobody against CPD1.

To further demonstrate specificity and the ability of oligobodies to perform as antibodies, an immunohistochemistry was performed. Mouse cerebella (C57BL/6J mice) were dissected at postnatal day 7, fixed in alcohol/acid (95% Ethanol: 5% acetic acid, 4 hours at -20°C), dehydrated, and embedded in paraffin. Five µm tissue slices were mounted in a silanized coverglass (Silane, Sigma Co., St. Louis, MO), deparaffinized, rehydrated and blocked using PBS-5%BSA for 1 hour. The slices were exposed to affinity-purified rabbit polyclonal antibodies overnight at 4 °C, rinsed twice with PBS, incubated with the secondary antibody (goat anti-rabbit peroxidase 1:1000) for one hour, and developed with DAB (Gibco BRL, Gaithersburg, MD). For immunohistochemistry using the monoclonal oligobody, a biotinylated-monoclonal oligobody was made by adding a 5'-biotinilated primer to the PCR reaction (5'-Biot-CTGCAGCCGCGGGGATCCT-3') (SEQ ID NO:8). After PCR amplification, the cDNA strands were separated by heat (94 °C, 5 minutes) and diluted ten times with PBS-5% BSA. After incubation of slides with PBS-5% BSA to block nonspecific sites, oligobodies were incubated with the tissue for 1 hour at RT and then developed with streptavidin-peroxidase (Promega Corp.). Control samples were made preincubating oligobodies with excess of blocking peptide overnight at 4 °C, followed by development as indicated above. As shown in Fig. 9, the results obtained by using affinity-purified polyclonal antibodies made in rabbits (Fig. 9A) were indistinguishable from those obtained with the monoclonal oligobody (Fig. 9 B). Controls made by pre-incubation of the monoclonal oligobody with the synthetic peptide corresponding to CPD1 showed no stain (Fig. 9 C). The stain for CPD1 was observed in both granule cells and in Purkinje cells, in which nuclear and plasma membrane staining is present. This constitutes the first example of a "histochemistry" made using a synthetic antibody-like reagent.

### Example 10: Western blot made using a chemically synthesized oligobody.

Using the sequence obtained as described in Example 5 (SEQ ID NO:7), a chemically synthesized oligobody was made against CPD1, using an oligonucleotide synthesizer (Beckman Oligo 1000 M). This reagent constitutes the first synthetic antibody-like reagent made so far entirely by chemical synthesi. As can be seen from the figure (Fig. 10), the synthetic oligobody recognizes very specifically CPD1 in a Western blot made with a mixture of all the proteins present in a cerebellar extract. Only one additional spot can be seen in the front of the run. This results also confirm the results described in the other examples, in which a PCR amplified product was used.

The present invention is distinguishable from previous art by the use of temporary targets, the "target switching" procedure, and most importantly, by the use of a selection based in specificity rather than on affinity. It is characterized also by the short number of steps required. The resulting oligobodies are identical in behavior and specificity to conventional antibodies as demonstrated for the first time by Western blots, immunohistochemistry and immunoprecipitations. Further, for the first time synthetic antibody-like reagents (oligobodies) can be obtained which demonstrate specific binding for both native and denatured proteins. These unique properties made these reagents suitable for procedures that normally use antibodies.

The foregoing description and examples have been set forth merely to illustrate that oligobodies behave identical to antibodies in their ability to recognize either native or denatured proteins, in a very specific way, and are not intended to be limiting since it is evident that oligobodies can replace antibodies in all applications involving recognition of native or denatured proteins, such as Western blots, immunoprecipitations, immunohistochemistry, radioimmunoassays, ELISA, pharmacological preparations for treatment of diseases, microarrays for diagnosis, etc.. Since modification of the disclosed embodiments incorporating the spirit and substance of the invention may occur to persons skilled in the art, the invention should be construed broadly to include all variations falling within the scope of the invention and appended claims, and equivalents thereof.

## Claims

1. Synthetic oligonucleotide-based antibody-like reagents or "oligobodies", comprising natural or derivatized oligonucleotides selected by a process based on high specificity rather than high affinity, which possess the unique ability to behave like natural polyclonal or monoclonal antibodies in their multiple applications, recognizing both native and/or denatured proteins in a very specific way.

2. A method of obtaining an oligobody comprising the steps of:
- providing a library of oligonucleotides having uniform, primer binding 5'-end and 3'-end sequences with random core sequences therebetween;
- incubating the oligonucleotide library with a synthetic temporary target corresponding to a portion of a final target molecule to which oligobodies are to be obtained for a time sufficient to permit binding of oligonucleotides from the library to said temporary target;
- selecting oligonucleotides which bind to the temporary target by
separating unbound oligonucleotides from the temporary target, and thereafter eluting and collecting oligonucleotides which bind to the temporary target during the incubation;
- amplifying the collected oligonucleotides;
- repeating the incubation with the temporary target, selecting and amplifying steps until detectable binding is obtained;
- thereafter incubating the collected and amplified oligonucleotides, which exhibit detectable binding, with a target mixture containing the final target molecule in a mixture with other molecules for a time sufficient to permit binding of oligonucleotides from the collected and amplified oligonucleotides to the final target molecule;
- selecting oligonucleotides which bind with increasing specificity to the final target by separating unbound oligonucleotides from the final target, and subsequently eluting and collecting oligonucleotides which bind with increased specificity to the final target;
- amplifying the oligonucleotides which bind with increased specificity collected from the final target; and
- repeating the incubation with a target mixture containing the final target molecule, selecting and amplifying steps until increases in binding specificity to the final target are no longer observed.

3. A method according to claim 2, wherein said final target molecule is a protein and said temporary target is a synthetic peptide corresponding to a fragment of said protein.

4. A method according to claim 3, wherein said synthetic peptide corresponds to an antigenic fragment of said protein.

5. A method according to claim 2, wherein said amplification is carried out by polymerase chain reaction using primers which bind to the uniform 5' and 3' end portions of the oligonucleotides from said oligonucleotide library.

6. A method according to claim 5, wherein said amplification is carried out by subjecting collected oligonucleotides which bind to the target to exponential PCR amplification; purifying the exponentially amplified oligonucieotides to remove truncated oligonucleotides and polymers; and thereafter subjecting the purified oligonucleotides to lineal PCR amplification using only one primer to obtain single stranded oligonucleotides.

7. A method according to claim 6, wherein said lineal PCR amplification is carried out in the presence of a label so that the amplified oligonucleotides are obtained in labeled form.

8. A method according to claim 7, wherein said label is selected from the group consisting of radiolabels, biotin and fluorescent labels.

9. A method according to claim 2, wherein said temporary target is bound to a solid matrix.

10. A method according to claim 9, wherein said solid matrix is a nitrocellulose paper.

11. A method according to claim 9, wherein said solid matrix is pre-treated to block non-specific binding sites.

12. A method according to claim 9, wherein said separating is carried out by washing unbound oligonucleotides from said solid matrix.

13. A method according to claim 2, wherein incubation is carried out only 2 or 3 times with the temporary target and only 2 or 3 times with the final target.

14. A method according to claim 13, wherein the total number of incubation cycles is not more than 5.

15. A method according to claim 2, wherein the oligonucleotides collected after increases in binding specificity are no longer observed, are ligated into a suitable vector, cloned, screened, purified and sequenced to obtain a monoclonal oligobody.

16. A method according to claim 15, further comprising producing increased amounts of the obtained monoclonal oligobody by PCR amplification, plasmid digestion or chemical synthesis (using natural or derivatized oligonucleotides).

17. A method according to claim 2, wherein said final target is a native protein, and the selecting of oligonucleotides which bind with increasing specificity to the final target is effected by a non-denaturing method which maintains the final target protein in a natural conformation selected from the group consisting of dot blots, liquid phase separation, immunohistochemistry, gel shifts and column separation.

18. An oligobody comprising oligonucleotides which bind specifically to a target protein, obtained by the method of claim 2.

19. A monoclonal oligobody comprising an oligonucleotide which binds specifically to a target protein, obtained by the method of claim 15.

20. An oligobody according to claim 18, wherein said oligonucleotides recognize both native and/or denatured proteins.

21. An article of manufacture comprising a solid matrix having attached thereto oligobodies comprised of oligonucleotides that bind specifically to a target protein.

22. Oligobodies comprising products made of nucleic acids of double or single strands.

23. Oligobodies comprising ribonucleic acids having natural or modified chemical groups.

24. Oligobodies comprising deoxyribonucleic acids, containing natural or modified chemical groups.

25. Oligobodies obtained by using as temporary target a fragment of the final target, which could be any polymer (protein, carbohydrate, oligonucleotide, etc.) or molecule of importance for biotechnology, research or clinics.

26. Oligobodies obtained using as temporary target a modified synthetic peptide (such as phosphorylated, isoprenylated, glycosylated).

27. A method of selecting a mixture of oligonucleotides each oligonucleotide being capable of specifically binding a target molecule, comprising the steps of:
(a) providing a library of oligonucleotides having uniform, primer binding 5'-end and 3'-end sequences with random core sequences therebetween;
(b) incubating the oligonucleotide library with a synthetic temporary target corresponding to a portion of a final target molecule for a time sufficient to permit binding of oligonucleotides from the library to said temporary target;
(c) selecting oligonucleotides which bind to the temporary target by separating unbound oligonucleotides from the temporary target, and thereafter eluting and collecting oligonucleotides which bind to the temporary target during the incubation;
(d) amplifying the collected oligonucleotides;
(e) repeating steps (b) to (d) until detectable binding is obtained;
(f) thereafter incubating the collected and amplified oligonucleotides, which exhibit detectable binding, with a target containing the final target molecule in a mixture with other molecules for a time sufficient to permit binding of oligonucleotides from the collected and amplified oligonucleotides to the final target molecule;
(g) selecting oligonucleotides which bind with increased specificity to the final target by separating unbound oligonucleotides from the final target, and subsequently eluting and collecting oligonucleotides which bind with increased specificity to the final target;
(h) collecting from the final target and amplifying the oligonucleotides which bind with increased specificity; and
(i) repeating steps (f) to (h) until increases in binding specificity to the final target are no longer observed.

28. A method for selecting an oligonucleotide capable of acting as a monoclonal antibody by specifically binding to a target, comprising the steps of:
(a) selecting a mixture of a oligonucleotides, according to the method defined in claim 27; and
(b) selecting an oligonucleotide from the mixture according to its capacity to bind to a target molecule.

29. A method according to claim 28, wherein step (b) comprises:
(b1) ligating each oligonucleotide from the mixture of oligonucleotides into a suitable vector;
(b2) cloning, screening, purifying and sequencing the oligonucleotides; and
(b3) selecting an oligonucleotide having a desired binding capacity using Western Blotting.

30. A mixture of oligonucleotides obtainable in accordance with the method as defined in claim 27 where each oligonucleotide is capable of specifically binding a target.

31. An oligonucleotide obtainable in accordance with the method as defined in claim 28 or claim 29, where the oligonucleotide is capable of acting as a monoclonal antibody by specifically binding a target.

32. An oligonucleotide or mixture of oligonucleotides according to claim 30 or 31 having a sequence length of from 30 to 200 nucleotides.

33. Use of an oligobody or a mixture of oligonucleotides or an oligonucleotide according to any one of claims 1, 18 to 20, 22 to 26, or 30 to 32 in a method of protein purification, affinity chromatography, library screening, cell sorting, or confocal microscopy.

34. An oligobody or a mixture of oligonucleotides or an oligonucleotide according to any one of claims 1, 18 to 20, 22 to 26, or 30 to 32 for use in medicine.

35. Use of an oligobody or a mixture of oligonucleotides or an oligonucleotide according to any one of claims 1, 18 to 20, 22 to 26, or 30 to 32 as a pharmaceutical carrier.

36. Use of an oligobody or a mixture of oligonucleotides or an oligonucleotide according to any one of claims 1, 18 to 20, 22 to 26, or 30 to 32 in the manufacture of an agent effective as a physiological inhibitor or activator.

37. A pharmaceutical composition comprising an oligobody or a mixture of oligonucleotides or an oligonucleotide according to any one of claims 1, 18 to 20, 22, to 26, or 30 to 32.

38. A kit for use in a diagnostic method containing an oligobody or a mixture of oligonucleotides or an oligonucleotide as defined in any one of claims 1, 18 to 20, 22 to 26, or 30 to 32.

39. A method for making an oligobody or an oligonucleotide comprising an oligobody or oligonucleotide as defined in any one of claims 18, 20, 22 to 26 or 31, comprising the step of constructing the oligobody or oligonucleotide in an oligonucleotide synthesizer.
